# EUROPEAN PATENT SPECIFICATION

(11) **EP 1 438 975 B1**
(45) Date of publication and mention of the grant of the patent: **16.11.2005**
(21) Application number: 03007468.6
(22) Date of filing: 04.04.2003
(51) Int. Cl.: A61L 15/28

(54) **Microbial-derived cellulose amorphous hydrogel wound dressing**
Wundauflage mit amorphem Hydrogel auf Basis mikrobiell veränderter Cellulose
Pansement à base d'un gel amorphé contenant un dérivé microbien de la cellulose

(30) Priority: 16.01.2003 US 345394; 16.01.2003 WO PCT/US03/01162
(43) Date of publication of application: 21.07.2004
(73) Proprietor: Xylos Corporation, Langhorne, PA 19047 (US)
(72) Inventor: Serafica, Gonzala, Langhorne, PA 19047 (US); Mormino, Richard, San Antonio, Texas 78258 (US); Hoon, Russel, Doylestown, PA 18901 (US)
(74) Representative: HOFFMANN EITLE

(56) References cited:
- US-A- 4 588 400
- US-A- 5 558 861
- US-A- 5 662 924
- US-A- 6 071 727
- US-A1- 2002 107 223

## Description

### Field of the Invention

The invention relates to a wound dressing comprising microbial-derived cellulose in an amorphous hydrogel form.

### Background of the Invention

There are numerous wound dressings that demonstrate effectiveness to aid in the healing of wounds. The components of these include various polymeric systems, cellulosic materials derived from plants and bacteria, and collagen. Each has its mode of action to assist the wound healing process. Many rely on either the donation of fluid to hydrate a wound surface and aid in removal of necrotic tissue through autolytic debridement or the absorption of excess fluid termed exudate.

Microbial-derived cellulose dressings are composed of bacterial cellulose and water. The processing of which, results in a dressing that possesses unique characteristics. Not only can it donate moisture which is associated with the dressing but its multi-layered three-dimensional structure, that distinguishes it from plant-derived cellulose, creates a material with a water-holding capacity up to 700 times its own dry weight, as described in U.S Patent 4,942,128. Microbial cellulose also demonstrates excellent wet tensile and compression strength. Lastly, by adjusting the cellulose to liquid ratio in processed microbial cellulose, the amount and rate of both fluid donation and absorption can be manipulated.

Because of its superior characteristics, use of microbial cellulose in the medical industry has been previously investigated. For example, U.S. Patent Nos. 4,588,400, 4,655,758 and 4,788,146 to Ring *et al.* disclose the possible use of microbial-derived cellulose in liquid-loaded medical pads. The patents to Ring *et al.* focus on using statically produced microbial cellulose pads loaded with various liquids and medicaments. These pads were detailed as well as the production and cleaning method to produce the starting cellulose material. Also described in these patents are examples detailing methods of fabrication of various pads, wherein the method involves a series of pressing and soaking steps to adjust the physical properties, mainly with respect to the liquid to cellulose ratio, to yield a desired product. As an example, these patents illustrate a highly hydrated pad (80 to 1 fluid to cellulose ratio) that is able to provide a cooling capability ideal for burn applications. In particular, the '146 patent describes the use of such liquid loaded pads as wet dressings for use as an ulcer dressing capable of providing moisture to the wound over an extended period of time. The same '146 patent mentions that the wet dressings described in the examples have the additional ability to absorb large quantities of fluid from the wound site when the dressing is applied in a less than saturated condition. However, the wound dressings of Ring *et al.* fail to mention a singular dressing having both the ability to be a source of moisture for wounds as well as the ability to absorb fluid. The Ring *et al.* patents also fail to describe the effective liquid to cellulose ratio to fabricate a dressing having the dual fluid handing capability. Furthermore, the Ring *et al.* patents do not describe microbial-derived cellulose wound dressings in an amorphous gel form.

Amorphous hydrogel dressings, for example IntraSite Gel (Smith & Nephew), differ from other dressings in their ability to add moisture to a dry wound and as such have been shown to be useful for debriding necrotic dry tissue found in chronic and burn wounds. Since these hydrogels have not been cross-linked and therefore do not take a fixed shape, they have been termed amorphous (Ovington, L. G., *Amorphous Gels Can Help Dry Escharic Wounds,* Wound Care Institute Newsletter, July/August 1997, Volume 2, No. 3).

Rhodes, in U.S. Patent 5,662,924, describes a wound dressing that contains a water-insoluble water swellable cross-linked cellulose derivative, water and a polyol. This dressing, in the form of an amorphous gel, is believed to enhance moisture penetration of necrotic tissue and thereby facilitate wound healing by speeding up debriding action.

### Summary of the Invention

It is an object of the present invention to provide a dressing which is particularly suitable for wound healing, particularly the healing of chronic wounds and dry wounds covered with dry necrotic tissue or eschar.

To solve the above object, the present invention provides a microbial-derived cellulose amorphous gel wound dressing having a cellulose content of 1.0% to 99% by weight, and a method of producing said dressing. The wound dressing is amorphous, i.e. the wound dressing does not retain its shape at a temperature of 25 °C and atmospheric pressure, such that the dressing, placed on a flat plate, flows, in contrast to the known cellulose pads. Moreover, the invention provides the use of these wound dressings for treating chronic wounds and burn wounds. The wound dressing of the present invention can be applied to the wounds using a syringe since the dressing shows the required flowability.

Other objects, features and advantages of the present invention will become apparent from the following detailed description.

The present inventors have developed a flowable cellulose-based gel wound dressing that possesses a novel fluid handling capability of absorption and donation. Surprisingly, production of a microbial-derived cellulose wound dressing in an amorphous gel form enhances the moisture donating aspect of the wound dressing relative to the unprocessed microbial cellulose starting film material. This fluid handling capability is an end result of the processed microbial cellulose that contains the proper cellulose content for the intended purpose. The resulting wound dressing can donate fluid if the wound surface is dry and found to be particularly useful for dry wounds covered with dry necrotic tissue or eschar. Here it acts to autolytically debride the wound: the necessary first step in healing of a chronic wound.

Surprisingly, the same dressing is also capable of absorbing fluid away from the exuding wound bed. Typically, chronic wounds such as venous ulcers tend to exude large amounts of fluids during the healing process. At this stage the dressing of the present invention is able to absorb the fluid exudate while maintaining a moist surface for epithelial cells to migrate. The epithelial migration is essential for eventually closing the wound.

Furthermore, the flowable nature of this material allows this dressing to fill areas that a pad cannot effectively treat. The amorphous gel dressing can be delivered to the entire wound bed surface. The intimate contact of the gel dressing with the entire wound surface further enhances the moisture donation and absorption quality of microbial-derived cellulose and thereby improves wound healing. When it is necessary to change the dressing the amorphous gel dressing can be easily removed without upsetting the newly forming tissue. Also, since it can be removed en bloc, the wound cleansing process, required for other gel dressing products, is greatly simplified.

### Detailed Description of the Preferred Embodiment

Unless otherwise specified, "a" or "an" means "one or more".

The preferred biosynthesized cellulose for the amorphous gel is produced by cellulose-producing organisms, such as *Acetobacter xylinum,* and is subjected to a series of chemical wash steps to render it non-pyrogenic. Once grown the typical processing uses hydroxide solutions at concentrations of 0.5-20% by weight. Preferably, sodium hydroxide is used at a concentration of not less than 1% by weight and most preferably 2% to 4% by weight in order to dissolve the cells. In addition, the present invention preferably provides hydrogen peroxide washing capable of whitening and sanitizing the non-pyrogenic films.

Cellulose pellicles are typically composed of greater than 98% water and from 0.2 to 2% cellulose by weight. Subsequent to chemical processing, the pellicles are wet milled to produce the amorphous gel form with a cellulose content roughly equivalent to that of the starting material but which can be adjusted to any desired concentration through the addition or removal of fluids. The amorphous gel wound dressing obtained from the milling and grinding of the intact microbial cellulose pellicles has a primary structure of ultra fine fibers that are known to be about 200 times finer than cotton fibers. The secondary structure, which is a non-woven pattern of interpenetrating cellulose fibers of virtually infinite length, is not completely disrupted. However, by the milling, the pattern is partially destroyed and the length of the fibers is reduced to an extent that a flowable dressing may be obtained.

Typical cellulose contents of the present invention ranges from 1.0% to about 99% cellulose by weight, preferably 2.5% to 65% by weight, more preferably 3.0% to 50% by weight and most preferably 3.5 % to 12% by weight. In an especially preferred embodiment, the cellulose content is 4% or 7% by weight.

The amorphous gel dressings of the invention can be used for donation of liquid to wounds as well as absorbing liquid from wounds. Typically, the microbial-derived cellulose dressing can donate between 40 to 85% of its liquid weight and can absorb between 10 to 50%, more preferably the dressing can donate 50 to 65% of its liquid weight and absorb 15 to 35 % of its weight in liquid.

The flowable nature of the wound dressing can be manipulated by the addition of an ingredient for flow modification. The type of ingredient for flow modification is not restricted, provided that it does not considerably affect the donation and absorption properties of the cellulose dressing and is suitable for providing a biocompatible product (biocompatibility defined in accordance with ISO 10993 for biomaterials used as medical devices). Such ingredients include polyols and other flow modification agents such as lecithin, aloe vera and Tween 80 (polysorbate 80; polyoxyethylenesorbitan monooleate). Preferred polyols are propylene glycol, glycerol, polyethylene glycol (preferably having a number-average molecular weight in the range of 300 to 1450) and sugars and sugar alcohols such as sorbitol, mannitol. Propylene glycol, glycerol and sugar alcohols are preferred.

The concentration of these additives in the microbial cellulose gel may vary from 1% to 50% by weight depending on the properties of the specific additive and on the desired flow characteristics of the resulting gel. The preferred concentration is in the range of 3 to 12 % by weight of the dressing, most preferably 9 to 11 % by weight and particularly 10 % by weight. The flow characteristics of the gel are preferably chosen to be suitable for application of the gel to the wound using a syringe.

Moreover, polymers can be included in the wound dressing to enhance its physical properties, particularly with respect to moisture absorption. Suitable polymers include polyethylene oxide (preferably being solid at 25° C), polyvinylpyrrolidone, and carboxymethylcellulose (CMC). The content of these polymers depends on the type of polymer used and is determined such that the donation properties, absorption properties and flow properties are optimized (see Example 5 below in this respect). The content may be in the range of up to 50 % by weight of the dressing. A more preferred range is 1 to 10 % by weight, particularly 1 to 4 % by weight.

The wound dressing may also contain bioactive substances, such as proteins, such as platelet derived growth factor (PDGF), epidermal growth factor (EGF), fibroblast growth factor (FGF), insulin-like growth factor (IGF), Transforming growth factor - beta (TGF-β), bone morphogenetic protein (BMP), vascular endothelial growth factor (VEGF), nerve growth factor (NGF), tumor angiogenesis factor (TAF), corticotropin releasing factor (CRF), interleukin-8 (IL-8), granulocyte-macrophage colony stimulating factor (GM-CSF), and other growth factors, and enzymes such as collagenase, papain and fibrinolysin desoxynuclease. Additionally the dressing may contain one or more active agents like antibiotics, such as bacitracin, polymyxin B, gentamicin, chloramphenicol, mupirocin, neomycin, silver sulfadiazine, gramicidin, and the like: topical anesthetics, such as lidocaine hydrochloride, benzocaine, dibucaine, tetracaine hydrochloride and the like: antifungal agents, such as clotrimazole, econazole, ketoconazole, miconazole, nystain, terbinafine, tolnaftate, undecylenic acid and the like; antiseptics and preservatives, such as polyhexamethylene biguanide, chlorhexidine digluconate, benzalkonium chloride, silver-based antimicrobials, copper-based antimicrobials and the like; antiviral agents, such as gentamycin sulfadiazine, dapsone, ampicillin, amphotericin B, silver halides, silver protein, colloidal silver, erythromycin and the like.

The dressing further contains water or an aqueous solvent such as isotonic saline. The water content is preferably in the range of 75 to 90 % by weight, more preferably 80 to 88 % by weight. In particularly preferred dressings the cellulose content is 3 to 10 % by weight (more preferred: 5 to 7 % by weight), the content of flow modifier is 8 to 12 % by weight (more preferred: 9 to 11 % by weight), and the water content is 78 to 89 % by weight (more preferred: 82 to 89 % by weight).

Compared to the intact microbial cellulose pellicles, the amorphous gel form can be formulated to enhance the donation and/or absorption characteristics of the gel. The content of microbial-derived cellulose present in the amorphous gel dressing can be manipulated depending upon the method of preparation and the eventual end use of the wound dressing.

The present invention also relates to the use of the wound dressings of the present invention for the treatment of wounds. In a preferred embodiment, chronic wounds or burns are treated with the inventive wound dressing. The method comprises applying the wound dressing to the wound site, filling the wound with the hydrogel dressing, and covering the wound with a secondary film layer. The dressing is preferably applied using a syringe. The frequency of changing the dressing is readily determined by one skilled in the art. In one embodiment, the dressing is changed twice daily to weekly.

The present invention will be illustrated through the following examples.

### Example 1

### 1. Production of Microbial Cellulose

In preparing the microbial cellulose amorphous gels of the invention, a microbial cellulose film is prepared. The film is prepared by using microorganisms such as *Acetobacter xylinum* which are cultured in a bioreactor containing a liquid nutrient medium at 30 degrees °C at an initial pH of 3-6. The medium is based on sucrose or other carbohydrates. Preferably, efficient film production is achieved using sucrose as a carbon source, ammonium salts as a nitrogen source, and corn steep liquor as nutrient source coupled with a proprietary trace elements supplement, which varies from the original Schramm & Hestrin medium (1954) used by those skilled in the art. This proprietary trace elements supplement is quantified in the following table:

### Trace Element Solution

| **Composition per Liter** | |
|---|---|
| EDTA Tetrasodium Salt | 570mg |
| FeSO₄ • 7H₂O | 200mg |
| ZnSO₄ • 7H₂O | 10mg |
| MnSO₄ • H₂O | 26mg |
| H₃BO₃ | 30mg |
| CoCl₃ • 6H₂O | 20mg |
| NiCl₂ **•** 6H₂O | 3.2mg |
| (NH₄)₆Mo₇O₁₄• 4H_{2kp[}O | 2.4mg |

Suitable bioreactors are selected which minimize evaporation and provide adequate oxygen-limiting conditions. Oxygen-limiting conditions may be varied depending upon the desired water content and thickness of the cellulose film. Generally, under oxygen-limited conditions, oxygen is present in an amount of 5%-21% of the total gas present at the air liquid interface. The bioreactor is composed of plastic box fitted with an airtight cover or a limited gas-permeable cover. Dimensions of the bioreactor can vary in configuration (cube or cylinder) depending on the shape and size of the cellulose film being produced. For example, a six inch diameter cylinder will produce a six inch diameter dressing, which can be used as is or cut to conform to the wound to be treated, prior to application.. By limiting the amount of oxygen in the fermentation medium, it is hypothesized that the *Acetobacter* utilizes the carbon available in the medium to produce more cellulose instead of using it for reproduction, thereby increasing the total yield of cellulose.

The fermentation process under static conditions was allowed to progress over for a period of 7- 30 days, during which the bacteria in the culture medium produced an intact cellulose pellicle containing the microorganisms. Depending on the desired thickness, which corresponds to a certain cellulose content per unit area, the fermentation is stopped and the pellicle is removed from the bioreactor. The excess medium contained in the pellicle is then removed by standard separation techniques such as compression or centrifugation prior to chemical cleaning and subsequent processing of the pellicle to yield a wound dressing with a cellulose to liquid ratio of 1:10 to 1:65. The raw cellulose pellicle has an increased sugar:cellulose yield of about 35%, compared to literature values of 10%. This increased yield coupled with an inexpensive nitrogen source resulted in a 40-fold reduction in production-cost of the raw cellulose film as compared to cellulose films produced according to the original Schramm & Hestrin medium [1954, J. Gen. Micro, 11:123-129].

### 2. Processing and Depyrogenation Procedures

After the cellulose film has been produced, the cells have to be removed from the cellulose pellicle for purification. Fontana et al. (1990, Appl. Biochem. Biotech, 24: 253-264) have described the cells as being apyrogenic, however, the unpurified cellulose pellicle has tested positive for pyrogens using the Limulus Amebocyte Lysate (LAL) test kit. This result necessitated the removal of the cells by chemical processing discussed here in order to pass the standard pyrogenicity test and qualify the microbial cellulose wound dressing as nonpyrogenic.

The cellulose pellicle is subjected to a series of chemical wash steps to convert the raw cellulose film into a medical grade and non-pyrogenic wound dressing material. Typical processing uses hydroxide solutions at concentrations of 1-20% by weight. Preferably, sodium hydroxide is used at a concentration of not less than 3% and most preferably 3% to 5% in order to dissolve the cells. In addition, the present invention provides hydrogen peroxide washing capable of bleaching and sterilizing the pyrogen-free films. Concentrations of 0.05% to 10% peroxide by weight are useful to effect whitening of the films. Preferably the amount of peroxide used in 0.1% to 0.5%. Other bleaching agents such as hypochlorite , hypobromite, and perborate may also be used.

Purification processes using various exposure times, concentrations and temperatures were conducted on the raw fermentation product. Processing times of 1-4 hours have been studied in conjunction with temperature variations of 30-100 degrees centigrade to optimize the process. The resulting films from each of the different operating conditions were tested for their respective pyrogen levels and physical characteristics. The process condition that yields a nonpyrogenic product in the least amount of time and lowest chemical concentration was then selected for economic reasons. The time involved in this process can be as much as 4 hours at about 90°C, preferably the time involved is 1-2 hours at 60°C to 80°C.

The amount of cellular debris left in the cellulose pad after processing may be measured by Limulus Amebocyte Lysate (LAL) test as outlined by the U.S. Food and Drug Administration (FDA) in 21 CFR10.90. The instant cleaning process outlined above provided a nonpyrogenic cellulose pad (<0.05 EU/ml). The allowable pyrogen content in Class I medical devices is 0.5 EU/ml (FDA LAL test Guideline). The steps of the LAL test are defined by the test kit manufacturer and can simply be followed to yield the pyrogen level in the cellulose film.

### Example 2

### Production of a Microbial Cellulose Amorphous Gel

This example presents a method for making an amorphous gel material from microbial cellulose sheets. The cellulose sheets were processed using the method described in Example 1 to remove pyrogens and other contaminants, and compressed to obtain a cellulose content of approximately four percent.

A 500g quantity of the processed and depyrogenated microbial cellulose was placed in a 1 gal blender (e.g. a Waring blender; alternatively, a dewatering press or a package filler may be used). To this 2500 ml of deionized water was added, and the mixture was processed using a 3 hp motor at high speed for 5 min to ensure consistency. The resulting mixture was decanted into a draining bin, and excess water was allowed to drain. After draining for 15 min, the mixture was pressed until the weight of the gel again reached 500 g.

Two 20 g samples of the gel were removed and dried to determine the cellulose content of the gel. The average dry weight was 0.85 g, indicating a cellulose content of 4.25% by weight

### Example 3

### Modification of Flow Properties

This example demonstrates how the viscosity and flow properties of a microbial cellulose amorphous gel can be modified through the addition of an ingredient for flow modification.

Amorphous gel was produced by the method described in example 1, and the final cellulose content was determined to be 3.95% by drying of 20g aliquots. Using this gel, nine 50g samples were prepared containing 0 to 40 percent propylene glycol by weight. The gels were mixed thoroughly to distribute the propylene glycol and then packed into identical 5cc disposable syringes with 1.5mm tip openings (manufactured by Becton Dickinson, Franklin Lakes, New Jersey; a 5 ml latex free syringe with slip tip).

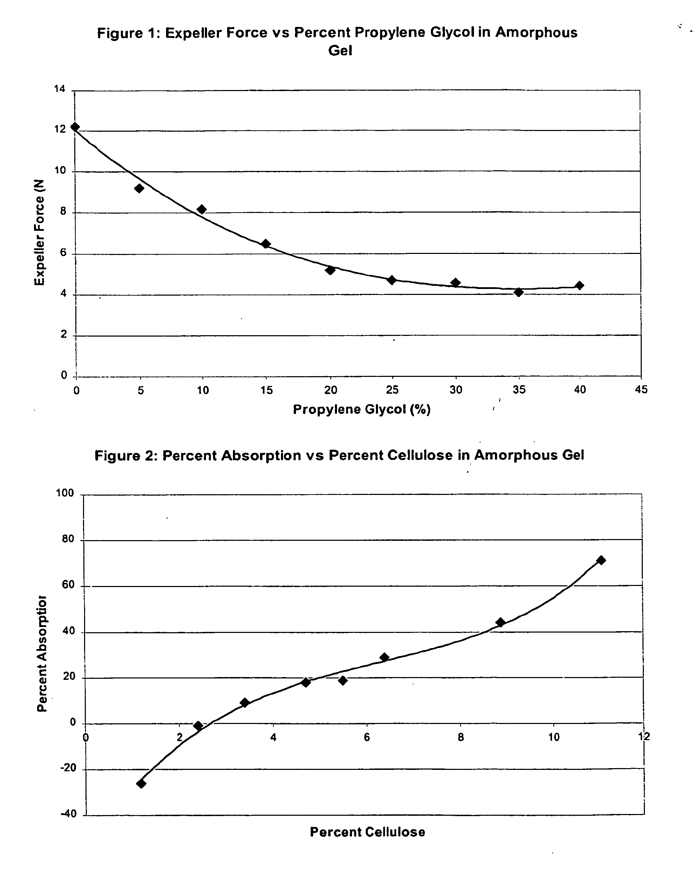

The minimum force required to discharge the material from the syringes was measured with a compact force gauge (i.e. a force measuring device) and was plotted versus the propylene glycol content. This image is shown in Figure 1. The discharge force initially dropped rapidly with the addition of the flow modifying agent, but the cumulative effect diminished as the concentration was increased. At around 25% propylene glycol the force leveled off at 4.5 N, with higher concentrations showing no discernible effect. An expeller force in the range of 10 to 4 N is preferred.

### Example 4

### Addition of Active Agents

This example shows how the properties of a microbial cellulose amorphous gel can be changed through the addition of active agents. The amorphous gel used for this example was produced using the method described in example 1.

The 500 g gel was divided in half. The first half was modified with the addition of polyhexamethylene biguanide (PHMB) in sufficient quantity to give a 0.25% concentration. The second half of the gel was kept unchanged. Both gels were sterilized by gamma irradiation at 30-35 kGy. The samples then underwent antimicrobial testing. 10 g samples of each gel were inoculated with 10⁵ cultures of either *Staphylococcus aureus* or *Esherichia coli* and incubated at 30°C. Organism populations were measured at time zero and again after 24 hr, and the totals of the PHMB-treated gel were compared with the untreated control.

### Results:

**Table 1**

| **Bacterial Inhibition by PHMB-Containing Amorphous Gel** | | | | |
|---|---|---|---|---|
| Sample | Population Count (cfu/ml) | | | |
| | Time 0 | | Time 24hr | |
| | S. aureus | E. coli | S. aureus | E. coli |
| | | | | |
| PHMB-treated | 1.5 x 10⁵ | 2.0 x 10⁵ | <10 | <10 |
| Untreated | 1.5 x 10⁵ | 2.0 x 10⁵ | 3.7 x 10² | 6.2 x 10⁴ |

The amorphous gel treated with 0.25% PHMB reduced the bacterial population of both species by 99.99%, whereas the untreated amorphous gel resulted in significantly less reduction.

### Example 5

### Preparation of an Amorphous Gel Wound Dressing

This example demonstrates the method of producing a wound dressing comprised of microbial cellulose amorphous gel. This dressing will have the ability to both donate moisture to or absorb moisture from a wound site, depending on the state of the wound.

Amorphous gel was produced following the method described in example 1. Using the 500 g gel as a base material and assuming the initial cellulose content to be 4%, eight samples were created ranging from 1 to 10 percent cellulose according to the following table:

**Table 2**

| **Composition of Amorphous Gel Samples** | | | | | |
|---|---|---|---|---|---|
| % Cellulose (assumed) | Mass of Gel (g) | Water (g) | | Total Weight | % Cellulose |
| | | Addition | Subtraction | | (actual*) |
| 1 | 12.5 | 37.5 | - | 50 | 1.17 |
| 2 | 25.0 | 25.0 | - | 50 | 2.41 |
| 3 | 37.5 | 12.5 | - | 50 | 3.42 |
| 4 | 50.0 | - | - | 50 | 4.71 |
| 5 | 62.5 | - | 12.5 | 50 | 5.48 |
| 6 | 75.0 | - | 25.0 | 50 | 6.39 |
| 8 | 100 | - | 50.0 | 50 | 8.87 |
| 10 | 125 | - | 75.0 | 50 | 11.1 |

| | | | | | |
|---|---|---|---|---|---|
| *A sample of each was dried to determine the exact cellulose content. | | | | | |

These samples were then tested for absorption from a saturated sponge and donation to a dry surface. For the absorption test, a 5 g sample of the gel was spread evenly over a 2in circular area on a sheet of filter paper. The paper was placed on top of a sponge sitting in a 0.9% saline bath at room temperature. The liquid level was maintained at the level of the sponge. Samples were removed after 24 hr and reweighed to determine the quantity of saline absorbed by the gel, and the absorption was reported as a percentage of the initial weight of the sample. Figure 2 shows the absorption profile for this set of amorphous gels. As can be seen, gels containing less than 3% cellulose lost weight during the test, indicating that moisture was donated to the wet sponge. The inflection point on the curve occurred at approximately 5.5% cellulose by weight, and increased rapidly from there as the cellulose content increased.

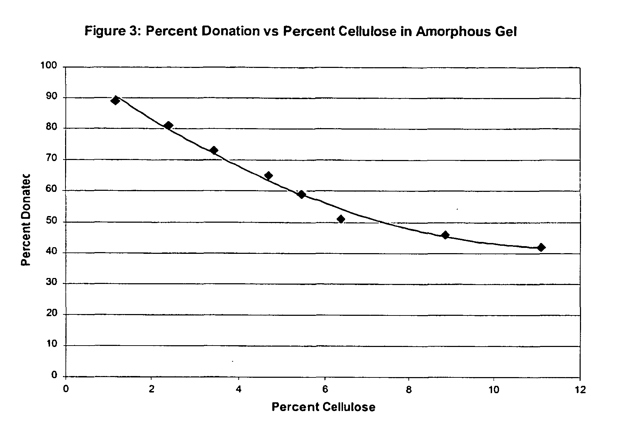

Donation testing was performed by spreading a 5 g sample of gel evenly over a 2 in diameter circular area on a 3 in x 3 in piece of pre-weighed smooth leather. Samples were removed after 2 hr and the leather was reweighed to determine the quantity of moisture donated to the dry surface. Donation results were reported as a percentage of the initial weight of the sample, and are shown graphically in Figure 3. Donation decreased nearly linearly up to 6% cellulose by weight, and then decreased more slowly up to the 11% by weight.

Using Figures 2 and 3, a wound dressing can be devised to accommodate both absorption and donation. In order to have measurable absorption, the gel would need to possess a minimum of 4% cellulose, and the gel would need less than 6% cellulose to donate significantly. Therefore, a wound dressing gel should contain between 4 and 6 percent cellulose to optimize the natural fluid handling ability of the microbial cellulose matrix.

## Claims

1. A microbial-derived cellulose amorphous gel wound dressing that does not retain its shape at temperature of 25°C and atmospheric pressure, having a cellulose content of 1.0% to 99% by weight.

2. The wound dressing of claim 1, having a cellulose content of 3 to 12 % by weight.

3. The wound dressing of any of the preceding claims, further comprising an ingredient for flow modification.

4. The wound dressing of claim 3, wherein the ingredient for flow modification is a polyol.

5. The wound dressing of claim 4, wherein said polyol is present in the dressing from 5 to 50 wt% and is selected from the group consisting of propylene glycol, glycerol, polyethylene glycol and sorbitol.

6. The wound dressing of any of the preceding claims, further comprising a preservative.

7. The wound dressing of claim 6, wherein the preservative is one or more of the following group: chlorhexidine digluconate, polyhexamethylene biguanide hydrochloride or silver compounds.

8. The wound dressing of claim 1, further comprising one or more active agents.

9. The wound dressing of any of the preceding claims, wherein the one or more active agents are selected from the group consisting of antimicrobials, antibiotics, antivirals, enzymes, proteins and growth factors.

10. The wound dressing of claim 9, wherein the antibiotic, antimicrobial or antiviral active agent is selected from the group consisting of bacitracin, polymixin B, gentamicin, chloramphenicol, mupirocin, neomycin, silver sulfadizine, gramicidin, ofloxicin, tetracycline, streptomycin, fluoroquinolones, ganciclovir, acyclovir, clindamycin, clortimazole, econazole, ketoconazole, miconazole, nystain, terbinafine, tolnaftate, undecylenic acid, gentamycin sulfadiazine, dapsone, ampicillin, amphotericin B, silver halides, silver protein, colloidal silver and erythromycin.

11. The wound dressing of claim 9, wherein the enzymes, proteins and growth factors are selected from the group consisting of collagenase, papain, fibrinolysin, desoxyribonuclease, platelet derived growth factor(PDGF),epidermal growth factor (EGF), acidic and basic fibroblast growth factors (FGF-1 and FGF-2), insulin-like growth factors 1+2(IGF-1 and IGF-2), vascular endothelial growth factor (VEGF), nerve growth factor (NGF), tumor angiogenesis factor (TAF), corticotropin releasing factor (CRF), interleukin-8 (IL-8), granulocyte-macrophage colony stimulating factor (GM-CSF), transforming growth factors alpha and beta (TGF - alpha and TGF-beta), bone morphogenetic protein (BMP), interferons, interleukins and albumin.

12. The wound dressing of any of the preceding claims, where the microbial-derived cellulose dressing donates 40 to 85% of its liquid weight and absorbs 10 to 50% of its weight.

13. The wound dressing of claim 12, wherein the microbial-derived cellulose dressing donates 50 to 65% of its liquid weight and absorbs 15 to 35% of its weight.

14. A method for preparing a microbial-derived cellulose amorphous gel wound dressing comprising:
production of a microbial cellulose pellicle;
isolation of a pellicle with a cellulose content by weight in the range of about 0.5 to about 1%; and
wet milling the pellicle to produce an amorphous gel with a cellulose content by weight of 1 % to 99 %.

15. The method as claimed in claim 14, wherein the microbial cellulose pellicle is obtained from *Acetobacter xylinum.*

16. A use of the wound dressing as defined in any of the claims 1 to 12 for the manufacture of a medicament useful in the treatment of chronic wounds.

## Patentansprüche

1. Amorpher Gelwundverband aus Cellulose mikrobiellen Ursprungs, der bei einer Temperatur von 25°C und Atmosphärendruck nicht formstabil ist, mit einem Cellulosegehalt von 1,0 bis 99 Gew.%.

2. Wundverband nach Anspruch 1 mit einem Cellulosegehalt von 3 bis 12 Gew.%.

3. Wundverband nach einem der vorhergehenden Ansprüche, weiterhin umfassend einen Inhaltsstoff zur Fliessmodifizierung.

4. Wundverband nach Anspruch 3, wobei der Inhaltsstoff für die Fliessmodifizierung ein Polyol ist.

5. Wundverband nach Anspruch 4, wobei das Polyol in dem Verband zu 5 bis 50 Gew.% vorliegt und ausgewählt wird aus der Gruppe bestehend aus Propylenglykol, Glycerin, Polyethylenglykol und Sorbitol.

6. Wundverband nach einem der vorhergehenden Ansprüche, weiterhin umfassend ein Konservierungsmittel.

7. Wundverband nach Anspruch 6, wobei das Konservierungsmittel ein oder mehrere Vertreter der folgenden Gruppe ist: Chlorhexidindigluconat, Polyhexamethylenbiguanid-hydrochlorid oder Silberverbindungen.

8. Wundverband nach Anspruch 1, weiterhin umfassend einen oder mehrere Wirkstoffe.

9. Wundverband nach einem der vorhergehenden Ansprüche, wobei der eine oder mehrere Wirkstoff (e) ausgewählt wird (werden) aus der Gruppe bestehend aus antimikrobiellen Mitteln, Antibiotika, antiviralen Mitteln, Enzymen, Proteinen und Wachstumsfaktoren.

10. Wundverband nach Anspruch 9, wobei das Antibiotikum, antimikrobielle Mittel oder antivirale Mittel ausgewählt wird aus der Gruppe bestehend aus Bacitracin, Polymixin B, Gentamicin, Chloramphenicol, Mupirocin, Neomycin, Silbersulfadizin, Gramicidin, Ofloxicin, Tetracyclin, Streptomycin, Fluorchinolonen, Ganciclovir, Acyclovir, Clindamycin, Clortimazol, Econazol, Ketoconazol, Miconazol, Nystain, Terbinafin, Tolnaftat, Undecylensäure, Gentamycinsulfadiazin, Dapson, Ampicillin, Amphotericin B, Silberhalogeniden, Silberprotein, kolloidalem Silber und Erythromycin.

11. Wundverband nach Anspruch 9, wobei die Enzyme, Proteine und Wachstumsfaktoren ausgewählt werden aus der Gruppe bestehend aus Kollagenase, Papain, Fibrinolysin, Desoxyribonuklease, von Plättchen abgeleitetem Wachstumsfaktor (PGDF), epidermem Wachstumsfaktor (EGF), sauren und basischen Fibroblastwachstumsfaktoren (FGF-1 und FGF-2), insulinartigen Wachstumsfaktoren 1+2 (IGF-1 und IGF-2), vaskulärem Endothel-Wachstumsfaktor (VEGF), Nervenwachstumsfaktor (NGF), Tumorangiogenesefaktor (TAF), Corticotropin-freisetzendem Faktor (CRF), Interleukin-8 (IL-8), Granulocyt-Macrophagenkoloniestimulierendem Faktor (GM-CSF), transformierenden Wachstumsfaktoren alpha und beta (TGF-alpha und TGF-beta), knochenmorphogenem Protein (BMP), Interferonen, Interleukinen und Albumin.

12. Wundverband nach einem der vorhergehenden Ansprüche, wobei der Verband aus Cellulose mikrobiellen Ursprungs 40 bis 85 % seines Flüssiggewichts abgibt und etwa 10 bis 50 % seines Gewichts absorbiert.

13. Wundverband nach Anspruch 12, wobei der Verband aus Cellulose mikrobiellen Ursprungs 50 bis 65 % seines Flüssiggewichts abgibt und etwa 15 bis 35 % seines Gewichts absorbiert.

14. Verfahren zur Herstellung eines amorphen Gelwundverbands aus Cellulose mikrobiellen Ursprungs, umfassend:
Herstellen einer mikrobiellen Cellulosepellicula;
Isolieren der Pellicula mit einem Cellulosegehalt im Bereich von etwa 0,5 bis etwa 1 Gew.%; und
Nassmahlen der Pellicula, um ein amorphes Gel mit einem Cellulosegehalt von 1 bis 99 Gew.% herzustellen.

15. Verfahren nach Anspruch 14, wobei das mikrobielle Cellulosepellicula aus Acetobacter xylinum erhalten wird.

16. Verwendung des Wundverbands nach einem der Ansprüche 1 bis 12 für die Herstellung eines Medikaments, das zur Behandlung chronischer Wunden nützlich ist.

## Revendications

1. Pansement en gel amorphe à base de cellulose d'origine microbienne qui ne conserve pas sa forme à une température de 25°C et à la pression atmosphérique, possédant une teneur en cellulose de 1,0 % à 99 % en poids.

2. Pansement selon la revendication 1, possédant une teneur en cellulose de 3 à 12 % en poids.

3. Pansement selon l'une quelconque des revendications précédentes, comprenant en outre un ingrédient pour modification de l'écoulement.

4. Pansement selon la revendication 3, dans lequel l'ingrédient pour modification de l'écoulement est un polyol.

5. Pansement selon la revendication 4, dans lequel ledit polyol est présent dans le pansement à 5 à 50 % en poids et est choisi dans le groupe constitué par le propylène glycol, le glycérol, le polyéthylène glycol et le sorbitol.

6. Pansement selon l'une quelconque des revendications précédentes, comprenant en outre un conservateur.

7. Pansement selon la revendication 6, dans lequel le conservateur est l'un ou plusieurs dans le groupe suivant : digluconate de chlorhexidine, chlorhydrate de polyhexaméthylène biguanide ou des composés de l'argent.

8. Pansement selon la revendication 1, comprenant en outre un ou plusieurs agents actifs.

9. Pansement selon l'une quelconque des revendications précédentes, dans lequel les un ou plusieurs agents actifs sont choisis dans le groupe constitué par les antimicrobiens, les antibiotiques, les antiviraux, les enzymes, les protéines et les facteurs de croissance.

10. Pansement selon la revendication 9, dans lequel l'agent antibiotique, antimicrobien ou antiviral actif est choisi dans le groupe constitué par la bacitracine, la polymixine B, la gentamycine, le chloramphénicol, la mupirocine, la néomycine, la sulfadiazine argentique, la gramicidine, l'ofloxacine, la tétracycline, la streptomycine, les fluoroquinolones, le ganciclovir, l'acyclovir, la clindamycine, le clotrimazole, l'éconazole, le kétoconazole, le miconazole, la nystatine, la terbinafine, le tolnaftate, l'acide undécylénique, la gentamycine sulfadiazine, la dapsone, l'ampicilline, l'amphotéricine B, les halogénures d'argent, le protéinate d'argent, l'argent colloidal et l'érythromycine.

11. Pansement selon la revendication 9, dans lequel les enzymes, les protéines et les facteurs de croissance sont choisis dans le groupe constitué par la collagénase, la papaïne, la fibrinolysine, la désoxyribonucléase, le facteur de croissance dérivé des plaquettes (PDGF), le facteur de croissance épidermique (EGF), les facteurs de croissance des fibroblastes acide et basique (FGF-1 et FGF-2), les facteurs de croissance insulinomimétiques 1 et 2 (IGF-1 et IGF-2), le facteur de croissance endothélial vasculaire (VEGF), le facteur de croissance nerveux (NGF), le facteur de l'angiogenèse tumorale (TAF), le facteur de libération de la corticotropine (CRF), l'interleukine-8 (IL-8), le facteur de stimulation des colonies de granulocytes et de macrophages (GM-CSF), les facteurs de croissance transformants alpha et bêta (TGF-alpha et TGF-bêta), la protéine morphogénétique osseuse (BMP), les interférons, les interleukines et l'albumine.

12. Pansement selon l'une quelconque des revendications précédentes, le pansement de cellulose d'origine microbienne donnant 40 à 85 % de son poids de liquide et absorbant 10 à 50 % de son poids.

13. Pansement selon la revendication 12, le pansement de cellulose d'origine microbienne donnant 50 à 65 % de son poids de liquide et absorbant 15 à 35 % de son poids.

14. Procédé de préparation d'un pansement en gel amorphe à base de cellulose d'origine microbienne comprenant :
la production d'une pellicule de cellulose microbienne ;
l'isolement d'une pellicule avec une teneur en poids de cellulose dans la fourchette d'environ 0,5 à environ 1 % ; et
le broyage humide de la pellicule pour produire un gel amorphe avec une teneur en poids de cellulose de 1 % à 99 %.

15. Procédé selon la revendication 14, dans lequel la pellicule de cellulose microbienne est obtenue à partir d'*Acetobacter xylinum.*

16. Utilisation du pansement tel que défini dans l'une quelconque des revendications 1 à 12 pour la fabrication d'un médicament utile dans le traitement de plaies chroniques.
